(19) Europäisches Patentamt
European Patent Office
Office européen des brevets

(11) **EP 0 890 117 B1**

(12) **EUROPÄISCHE PATENTSCHRIFT**

(45) Veröffentlichungstag und Bekanntmachung des Hinweises auf die Patenterteilung:
**02.01.2002   Patentblatt 2002/01**

(51) Int Cl.$^7$: **G01V 15/00**, G01V 3/10

(21) Anmeldenummer: **97908104.9**

(86) Internationale Anmeldenummer:
**PCT/CH97/00132**

(22) Anmeldetag: **27.03.1997**

(87) Internationale Veröffentlichungsnummer:
**WO 97/36192 (02.10.1997 Gazette 1997/42)**

(54) **VORRICHTUNG UND VERFAHREN ZUR POSITIONSBESTIMMUNG**

DEVICE AND PROCESS FOR DETERMINING POSITION

DISPOSITIF ET PROCEDE DE DETERMINATON DE POSITION

(84) Benannte Vertragsstaaten:
**AT BE CH DE DK FR GB IT LI NL SE**

(30) Priorität: **27.03.1996  CH 79796**

(43) Veröffentlichungstag der Anmeldung:
**13.01.1999   Patentblatt 1999/02**

(73) Patentinhaber: **MedNetix AG**
**5232 Villigen (CH)**

(72) Erfinder:
 • **BOKSBERGER, Hans, Ulrich**
 **CH-5225 Oberbözberg (CH)**
 • **GREUTER, Urs**
 **CH-5400 Baden (CH)**

 • **KIRSCH, Stefan**
 **CH-5203 Würenlingen (CH)**
 • **SEILER, Paul, Gerhard**
 **CH-5234 Villigen (CH)**
 • **SCHILLING, Christian**
 **CH-5303 Würenlingen (DE)**

(74) Vertreter: **Troesch Scheidegger Werner AG**
**Patentanwälte Postfach**
**8032 Zürich (CH)**

(56) Entgegenhaltungen:
 EP-A- 0 091 577     EP-A- 0 364 045
 EP-A- 0 425 319     WO-A-94/04938
 US-A- 4 317 078

## Beschreibung

Die vorliegende Erfindung betrifft eine Vorrichtung nach dem Oberbegriff der Patentansprüche 1 und 2, eine Verwendung derselben sowie ein Verfahren nach dem Oberbegriff der Patentansprüche 15 und 16.

Bei zahlreichen technischen und medizinischen Verfahren sind Informationen über die Position eines Objektes von grösster Bedeutung. Währenddem in der Medizin die Position von einzelnen Gewebeteilen - beispielsweise eines Tumors, der zur Zerstörung oder zur Wachstumsbegrenzung bestrahlt werden soll - bestimmt werden muss, ist die Positionserfassung zur Eingabe in ein Computersystem, beispielsweise für "Cyber Space"- Anwendungen, von allgemeiner Bedeutung. Eine solche Positionserfassungs-bzw. Positionseingabeeinheit wird in diesen Anwendungen auch etwa als dreidimensionale Maus bezeichnet. In diesem Zusammenhang sei auf die Druckschriften US-4 737 794, US-4 945 305 und US-5 453 686 verwiesen.

Eine medizinischen Anwendungen besteht - wie erwähnt - in der Behandlung von Tumoren im menschlichen Körper, wobei der Tumor mittels Photonen- oder in Spezialfällen auch mit Protonenstrahlen bestrahlt wird. Dabei ist das Ziel einer derartigen Strahlenbehandlung, dass lediglich der den Tumor bildende Gewebeteil bestrahlt wird. Das den Tumor umgebende Gewebe soll dabei so gering wie nur möglich geschädigt werden. Diese Forderung versucht man dadurch zu erreichen, indem die Dosisverteilung der applizierten Strahlung möglichst genau dem Tumorvolumen angepasst wird bzw. auf den Ort des Tumors begrenzt ist.

Verschiedene Methoden sind sowohl für die Photonen- als auch für die Protonenbestrahlung bekannt, wobei zum Teil erhebliche Qualitätsunterschiede zwischen den verschiedenen Methoden bestehen. Bei all diesen bekannten Methoden wird-unter Vermeidung einer Schädigung von gesundem Gewebe - vorausgesetzt, dass eine einmal diagnostizierte Tumorposition über den Behandlungszeitraum konstant bleibt.

Bei der Behandlung von ortsfesten Tumoren wurden teilweise beachtliche Erfolge erzielt. So hat sich insbesondere die Behandlung von Augenhintergrundmelanomen mit Protonenstrahlen als äusserst erfolgreich erwiesen.

Demgegenüber sind Tumore im Brust- und Bauchbereich im allgemeinen nicht ortsfest. Ihre Position wird vielmehr durch natürliche Bewegungsabläufe, wie beispielsweise durch die Atmung, die Herzkontraktionen, die Peristaltik, usw., dauernd verändert.

Sollen ähnliche Behandlungserfolge wie bei ortsfesten Tumoren erreicht werden, so muss die Position des Tumors während der Bestrahlung genau bekannt sein.

In einem Aufsatz von K. Ohara et al. mit dem Titel "Irradiation Synchronized with Respiration Gate" (International Journal on Radiation Oncology Biology Physics, 1989, Vol. 17, Seiten 853 bis 857) wurde aus diesem Grund eine Echtzeitsimulation der Tumorposition vorgeschlagen, wobei als Grundlage der Simulation die Verformung der Körperoberfläche, insbesondere die Verformung durch die Atembewegung, verwendet wurde. Die Methode weist jedoch Ungenauigkeiten auf, da es sich einerseits nicht um eine direkte Messung der Tumorposition sondern lediglich um eine indirekte Messung handelt, und da anderseits die weiteren positionsbestimmenden Faktoren-wie Herzkontraktion und Darmbewegung - nicht berücksichtigt werden.

Aus der WO 94/04938 ist eine Vorrichtung zur Positionsbestimmung bekannt, wobei mehrere Sendeeinheiten und eine Empfangseinheit vorgesehen sind. Mit Hilfe der Sendeeinheiten werden - zeitlich gestaffelt - Dipolfelder erzeugt, die mit der Empfangseinheit empfangen werden. Aufgrund der empfangenen Signale wird die Position der Empfangseinheit bestimmt.

Die bekannte Lehre weist den Nachteil auf, dass die Messgenauigkeit insbesondere bei kleinen Abmessungen für die Empfangseinheit ungenügend ist.

Der vorliegenden Erfindung liegt daher die Aufgabe zugrunde, eine Vorrichtung anzugeben, bei der die Position von Objekten genauer bestimmt werden kann.

Diese Aufgabe wird durch die im kennzeichnenden Teil der Patentansprüche 1 und 2 angegebenen Massnahmen und die in dem Patentansprüchen 15 und 16 angegebenen Verfahren gelöst. Vorteilhafte Ausgestaltungen der Erfindung und eine Verwendung derselben sind in weiteren Ansprüchen angegeben.

Mit Hilfe der erfindungsgemässen Vorrichtung kann die Position eines Objektes im Raum äusserst genau bestimmt werden. Darüber hinaus kann die Positionsbestimmung ohne direkte Verbindung zum Objekt erfolgen.

Wird am Tumor oder an einem sich in der Nähe des Tumors befindenden Gewebeteils eine miniaturisierte, Signale ausstrahlende Sendeeinheit befestigt, so dann durch den Empfang dieser Signale ausserhalb des Körpers mit Hilfe von Empfangseinheiten die Position des Tumors jederzeit genau berechnet werden. In analoger Weise kann die Positionbestimmung auch dadurch vorgenommen werden, dass die Empfangseinheit im bzw. beim Tumor und die Sendeeinheit bzw. Sendeeinheiten ausserhalb des Körpers plaziert werden. Die letztgenannte Anordnung hat darüber hinaus den Vorteil, dass der Körper einer geringeren Sendeleistung und damit einer geringeren thermischen Belastung ausgesetzt wird als bei der erst genannten Methode. Zudem gestaltet sich die Übertragung der beim Objekt, d.h. dem Tumor, gemessenen Signale nach aussen bei der letztgenannten Anordnung viel einfacher, da für diese Übertragung eine geringere Übertragungsleistung zur Verfügung gestellt werden muss als bei der erst genannten Methode.

Bei der medizinischen Anwendung wird die in der Nähe des Objektes, d.h. des Tumor, positionierte Einheit, falls dies notwendig ist, durch einen chirurgischen Eingriff in den Körper des Patienten implantiert. Bei der erst genannten Anordnung wird zum Aussenden von Signalen durch die Sendeeinheit Energie benötigt, die entweder über ein äusseres Feld an die Sendeeinheit oder über eine Drahtverbindung zwischen einer Generatoreinheit und der Sendeeinheit übertragen. In einer weiteren Ausführungsform der Erfindung weist die Sendeeinheit einen eigenen Energiespeicher auf, der demzufolge mitimplantiert wird.

Bei der zweitgenannten Anordnung gelten die obigen Aussagen sinngemäss, d.h., dass die durch die Empfangseinheit bzw. Empfangseinheiten empfangenen Signale über Drahtverbindungen nach aussen übertragen werden. Allerdings wird - wie erwähnt - bei dieser Realisierungsform für die Übertragung der Messsignale weniger Energie benötigt.

Die Erfindung wird nachfolgend anhand von Zeichnungen beispielsweise näher erläutert. Dabei zeigt

Fig. 1    ein vereinfachtes Funktionsblockschaltbild der erfindungsgemässen Vorrichtung,

Fig. 2    einen Aufbau einer als Empfangseinheit in der erfindungsgemässen Vorrichtung verwendeten Induktionsspule und

Fig. 3    eine weitere Ausführungsform der Induktionsspule gemäss Fig. 2.

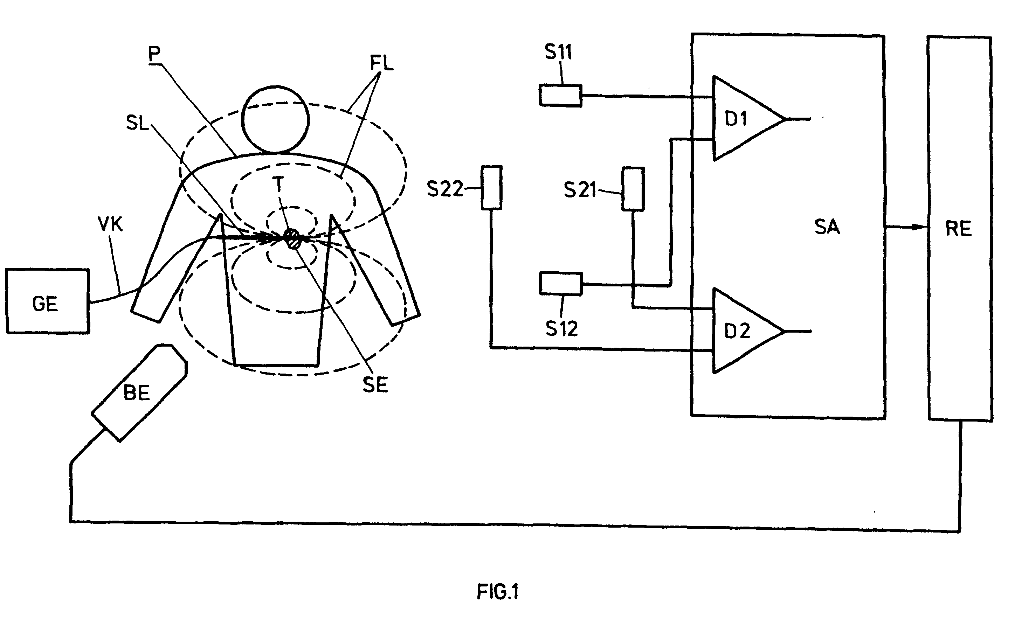

Fig. 1 zeigt einen menschlichen Körper P eines mit der erfindungsgemässen Vorrichtung zu behandelnden Patienten. Dabei besteht die erfindungsgemässe Vorrichtung aus einer Sendeeinheit SE, einer Generatoreinheit GE, einem Schlauch SL, Empfangseinheiten S11 bis S22, einer Signalaufbereitungseinheit SA, einer Recheneinheit RE und einer Bestrahlungseinheit BE.

Wie eingangs erwähnt ist die Kenntnis der genauen Position eines zu bestrahlenden Gewebeteils T, beispielsweise eines Tumors, unabdingbare Voraussetzung für eine maximale Schonung des an den Tumor angrenzenden gesunden Gewebes. Für diese Positionsbestimmung wird erfindungsgemäss die miniaturisierte Sendeeinheit SE möglichst nahe, vorzugsweise unmittelbar beim Tumor positioniert, damit die Sendeeinheit SE möglichst alle Bewegungen, die der zu bestrahlende Gewebeteil T erfährt, ebenfalls mitmacht.

Eine Möglichkeit, die Sendeeinheit SE am gewünschten Ort im Körper zu positionieren, besteht in der Verwendung einer Punktationshohlnadel, mit deren Hilfe der Schlauch SL von der Körperoberfläche zum Gewebeteil T geführt wird. Durch diesen Schlauch SL wird die Sendeeinheit SE in den Gewebeteil T bzw. in der Nähe des Gewebeteils T gebracht.

Befindet sich der zu bestrahlende Gewebeteil T an der Körperoberfläche oder in der Nähe eines natürlichen Körperhohlraumes, so ist die Sendeeinheit SE selbstverständlich an der Körperfläche zu fixieren bzw.

die Sendeeinheit ist, wenn möglich, durch eine natürliche Körperöffnung in den betreffenden Körperhohlraum einzuführen, ohne dass Gewebe durchdrungen werden muss, wie dies beispielsweise bei der Verwendung einer Punktationshohlnadel der Fall ist.

Falls die erfindungsgemässe Positionsbestimmung dazu verwendet wird, um eine Bestrahlung eines Gewebeteils T, beispielsweise eines Tumors, vornehmen zu können, so ist die Recheneinheit RE mit der Bestrahlungseinheit BE wirkverbunden. Damit kann die Bestrahlungseinheit BE aufgrund der Positionsangaben der Recheneinheit RE präzise auf den Gewebeteil T einwirken, wobei zwei grundsätzliche Möglichkeiten des Bestrahlungsvorganges denkbar sind:

Einerseits ist denkbar, dass ein Zielbereich, in dem die Strahlen ihre volle Wirkung entfalten, dem sich bewegenden und bestrahlenden Gewebeteil T nachgeführt wird oder, anderseits, dass die Bestrahlung lediglich dann vorgenommen wird, wenn sich der Gewebeteil T im fix vorgegebenen Zielbereich befindet.

In der bereits erwähnten und in Fig. 1 dargestellten Ausführungsform der Erfindung erfolgt die Energieversorgung der Sendeeinheit SE über ein durch den Schlauch SL geführtes Verbindungskabel VK, das an die Generatoreinheit GE auf der einen Seite und an die Sendeeinheit SE auf der anderen Seite angeschlossen ist.

Denkbar ist jedoch auch, dass die Sendeeinheit SE einen Energiespeicher, beispielsweise in Form einer Batterie, aufweist oder dass die Sendeeinheit SE von einem durch die Generatoreinheit GE erzeugten elektromagnetischen Feld erregt wird. Diese beiden Ausführungsformen haben den Vorteil, dass keine Verbindungskabel VK zwischen der Generatoreinheit GE und der Sendeeinheit SE notwendig sind, womit ein von der Sendeeinheit SE ausgestrahltes Feld, das zur Positionsbestimmung verwendet wird, nicht gestört wird. Zudem kann die Sendeeinheit SE zwischen einzelnen Behandlungen im Körper belassen werden. Nachteilig ist jedoch - insbesondere bei der Ausführungsform mit dem in der Sendeeinheit SE integrierten Energiespeicher - die daraus resultierende, grössere Sendeeinheit SE, was insbesondere bei einer Implantation in den Körper störend sein kann.

Die Sendeeinheit SE wird dazu verwendet, ein elektromagnetisches Feld, in Fig. 1 dargestellt durch einzelne Feldlinien FL, aufzubauen, das von den Empfangseinheiten S11 bis S22, die vorzugsweise ausserhalb des Körpers P angeordnet sind, empfangen werden kann. Zwar stellt ein menschlicher Körper P hinsichtlich der Ausbreitungseigenschaften von elektromagnetischen Wellen in den verschiedenen Geweben ein sehr inhomogenes Medium dar, jedoch ist die Beeinflussung eines den Körper P durchdringenden Magnetfeldes vernachlässigbar. Aus diesem Grund besteht die bevorzugte Ausführungsform der im Körper P plazierten Sendeeinheit SE aus einer miniaturisierten Spule, wobei ein von dieser Spule ausgehendes Magnetfeld einem magnetischen Dipol entspricht. Kennt man das magnetische Moment und die Lage eines Dipols im Raum, so ist die Stärke des Magnetfeldes in jedem Punkt im Raum berechenbar, wobei die Werte für die Magnetfeldstärke durch die drei kartesischen Koordinaten x, y, z, den Polarwinkel $\phi$ und das Azimut $\theta$ des Dipols eindeutig bestimmt sind. Das magnetische Moment eines Dipols kann berechnet oder durch geeignete Messungen bestimmt werden.

Die Position des Dipols im Raum, wie sie in der vorliegenden Anwendung benötigt wird, stellt das inverse Problem dar, nämlich ausgehend von den Messungen der Feldstärken des vom Dipol ausgestrahlten Feldes wird die Position des Dipols ermittelt.

Eine vollständige Positionsbestimmung erfordert die Kenntnis des magnetischen Moments der Sendeeinheit SE bzw. der in der Sendeeinheit SE enthaltenen Sendespule und die Messung von mindestens fünf linearen unabhängigen Ableitungen ihres Magnetfeldes.

Eine mögliche Methode zur Bestimmung der Position eines magnetischen Dipols im Raum ist im Aufsatz von W. M Wynn et al. mit dem Titel "Advanced Superconducting Gradiometer/Magnetometer Arrays and a Novel Signal Processing Technique" (IEEE Transactions of Magnetics, Vol. MAG-11, No. 2, März 1975, Seiten 701 bis 707) beschrieben. Allerdings wird bei dieser bekannten Methode zur Positionsbestimmung von einem Dipol ausgegangen, bei dem das magnetische Moment unbekannt ist. Aus diesem Grund muss zusätzlich zu den Feldableitungen noch eine Komponente des Magnetfeldes gemessen werden.

Bei der Anwendung dieser Methode auf die erfindungsgemässe Lehre kann das magnetische Moment jedoch in einer von den Messungen zur Positionsbestimmung unabhängigen Messung erhalten werden, d.h. auf die Messung der Feldkomponente während der Positionsbestimmung kann verzichtet werden.

Zur Messung der Feldableitungen (Feldgradienten) sind die Empfangseinheiten S11 bis S22 vorgesehen, wobei jeweils zwei dieser Empfangseinheiten, nämlich S11 und S12 bzw. S21 und S22, zur Bestimmung einer Feldableitung verwendet werden. Der Einfachheit halber sind in Fig. 1 lediglich die vier Empfangseinheiten S11 bis S22 dargestellt. Tatsächlich sind insgesamt zehn Empfangseinheiten notwendig, damit die fünf Variablen x, y, z, $\phi$ und $\theta$ eindeutig bestimmt werden können. Zur Überprüfung der gemessenen Werte ist jedoch auch vorgesehen, mehr als zehn Empfangseinheiten einzusetzen, womit redundante Informationen erhalten werden, aufgrund deren die Genauigkeit der Messungen eingeschätzt werden kann.

Als Empfangseinheit S11 bis S22 werden vorzugsweise Induktionsspulen verwendet. Die Induktionsspulen integrieren den magnetischen Fluss innerhalb ihres Volumens. Aus diesem magnetischen Fluss lässt sich dann die mittlere magnetische Feldstärke im Spulenvolumen bestimmen. Für die Positionsbestimmung ist man jedoch an der Feldstärke in einem Punkt im Raum interessiert.

Aus der Druckschrift mit dem Titel "Experimental Methods in Magnetism" von E. P. Wohlfarth (Band 2, Kapitel 1, Seiten 2 bis 7) ist bekannt, dass, wenn die Spulendimensionen geeignet gewählt werden, der Messwert, den die Spule liefert, mit nur geringen Abweichungen dem Wert der magnetischen Feldstärke, und zwar im Zentrum der Spule, entspricht. Dies kann insbesondere dann erwartet werden, wenn das Verhältnis von Länge zu Durchmesser der verwendeten Induktionsspule nach folgender Formel berechnet wird:

$$\frac{\zeta}{\rho_2} = \frac{3}{\sqrt{20}} \cdot \sqrt{\frac{1 - \gamma^5}{1 - \gamma^3}}$$

wobei

$$\gamma = \frac{\rho_1}{\rho_2}$$

und $\rho_1$, $\rho_2$ der innere bzw. der äussere Durchmesser der Induktionsspule ist. Es wurde unlängst gezeigt, dass - wenn $\gamma < 0.3$ ist - ein Anteil vierter Ordnung kleiner als $2 \times 10^{-3}$ ist und somit einen kleineren Einfluss ausübt als die magnetische Induktion $B_z$ entlang der Symmetrieachse der Induktionsspule.

Wie bereits erwähnt wurde, werden jeweils zwei der Empfangseinheiten S11 bis S22 zur Bestimmung der Ableitungen (Gradienten) des Magnetfeldes zusammen geschaltet. Dazu sind in der Signalaufbereitungseinheit SA Verstärkungseinheiten D1 und D2 mit je zwei Eingängen vorgesehen, wobei an jedem Eingang eine Empfangseinheit S11 bis S22 bzw. eine Induktionsspule angeschlossen wird. Es wird erneut darauf hingewiesen, dass für eine Positionsbestimmung im Raum eine entsprechend grössere Anzahl an Verstärkungseinheiten vorhanden sein müssen, als dies Fig. 1 entnommen werden kann, denn in Fig. 1 wurden der Übersichtlichkeit halber lediglich zwei Verstärkungseinheiten D1 und D2 bzw. lediglich vier Empfangseinheiten S11 bis S22 dargestellt.

In den Verstärkungseinheiten D1 und D2 wird die Magnetfelddifferenz zwischen den beiden an den Eingängen anstehenden Signalwerten gebildet. Diese Magnetfelddifferenz wird der Ableitung des Magnetfeldes näherungsweise gleichgesetzt.

Aufgrund der Miniaturisierung, insbesondere einer als Spule ausgebildeten Sendeeinheit SE, ist die erzeugte Magnetfeldstärke sehr klein. Damit stellen andere Magnetfeldquellen, wie sie in nicht speziell abgeschirmten Räumen vorkommen können, ein Problem dar. Aus diesem Grund wird in der Signalaufbereitungseinheit SA eine schmalbandige Filterung und/oder eine phasenempfindliche Verstärkung der in den Verstärkungseinheiten D1 und D2 erhaltenen Signalwerte vorgenommen. Damit kann ein grosser Teil der unerwünschten Signalanteile, inklusive Rauschen, eliminiert werden. Ferner tragen auch die zur Bestimmung der Ableitungen des Magnetfeldes (Gradienten) benötigten Differenzbildungen in den Verstärkungseinheiten D1 und D2 zur Reduktion von Störeinflüssen bei.

Die Induktionsspulen müssen sehr homogen und reproduzierbar gewickelt werden, damit von der in der Spule induzierten Spannung mit hoher Genauigkeit auf die Magnetfeldstärke geschlossen werden kann. Die Homogenität und Reproduzierbarkeit sind mit herkömmlichen Wicklungen aus Kupferrunddraht nur äusserst schwer zu erreichen. Aus diesem Grund werden nachstehend zwei weitere Möglichkeiten zur Realisierung von Induktionsspulen vorgeschlagen, bei denen die vorstehend genannten Nachteile vermieden werden.

Zunächst sei die bereits eingangs erwähnte erfindungsgemässe Vorrichtung erläutert, die sich von der erst genannten dadurch unterscheidet, dass Sende- und Empfangseinheiten ausgetauscht sind, womit ein sogenanntes Differenzfeld aufgrund der zur Messung der Feldableitung zusammen geschalteten Spulen, die nunmehr von einem vorgegeben Strom durchflossen werden, entsteht. Somit ist bei dieser Ausführungsvariante die Empfangseinheit bzw. die Empfangseinheiten im oder in der Nähe des Objektes T - d.h. des Tumors - und die Sendeeinheit bzw. die Sendeeinheiten ausserhalb des Raumes K - d.h. des Körpers - positioniert. Diese Ausführungsform hat den Vorteil, dass die ausserhalb des Körpers K liegenden Sendeeinheiten höher liegende Leistungsgrenzwerte aufweisen können als bei der erst genannten Variante. Dadurch wird bei der Empfangseinheit ein erheblich verbesserter Signal-Rausch-Abstand erreicht, womit die Anforderungen an die die Messsignale verarbeitende Recheneinheit geringer sind und womit die Messresultate genauer werden. Darüber hinaus benötigt die beim Objekt plazierte Empfangseinheit viel geringere Energiemengen zur Übertragung der Messsignale nach aussen, womit in den meisten Fällen eine Batterie-gestützte oder auf einem Transponderprinzip beruhende Empfangseinheit dazu ausreichend ist.

Um die Genauigkeit der Messsignale weiter zu verbessern, sind mehrere Kalibrationsspulen mit bekannten Positionen vorgesehen, aufgrund derer Störgrössen erfasst werden, die bei der Positionsbestimmung in korrigierender Weise verwendet werden.

Bei dieser zweiten Ausführungsvariante ist bei der Positionsbestimmung sowohl ein Zeit- als auch ein Frequenzmultiplexverfahren anwendbar: Beim Zeitmultiplexverfahren sendet jeweils nur eine Sendeeinheit - falls ein Differenzfeld gesendet wird, die zwei entsprechenden Sendeeinheiten S11 und S12 bzw. S21 und S22 - in einem definierten Zeitabschnitt. Beim Frequenzmultiplexverfahren hingegen senden alle Sendeeinheiten gleichzeitig, allerdings mit definierten, sich von anderen unterscheidenden Frequenzen.

In der vorstehend genannten zweiten Ausführungsform der Erfindung wird von der in Fig. 1 dargestellten Konfiguration ausgegangen, wobei Sende- und Empfangsort ausgetauscht wurden. Denkbar ist jedoch auch eine Ausführungsvariante, bei der in der Empfangseinheit SE beispielsweise zwei Spulen vorgesehen sind, mit denen entweder Absolutwerte oder Feldableitungen gemessen werden. Entsprechendes gilt auch für die Sendeeinheiten S11 bis S22, die je als Spule oder als Spulenpaar ausgebildet sein können.

Für die Bestimmung der genauen Position sei auf den Aufsatz von S. Kirsch et. al. mit dem Titel "Real Time Tracking of Tumor Positions for Precision Irradiation" (Proceedings of the Second Symposium on Hadrontherapy, September 9-13, 1996) hingewiesen.

Im folgenden wird auf zwei weitere Möglichkeiten zur Realisierung von Induktionspulen eingegangen, die es ermöglichen, die in die Spulen induzierten Spannungen mit hoher Genauigkeit zu bestimmen:

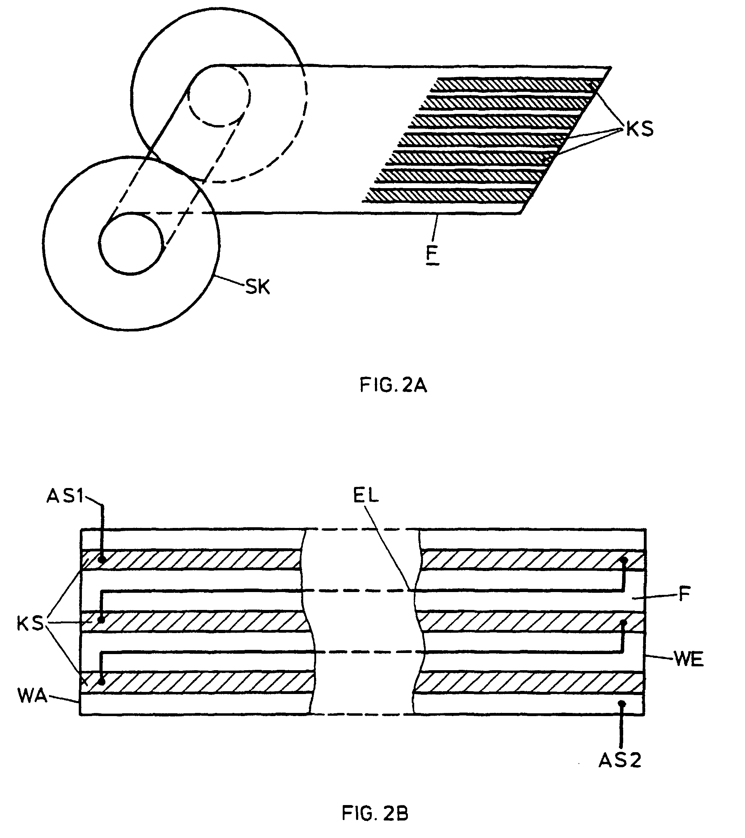

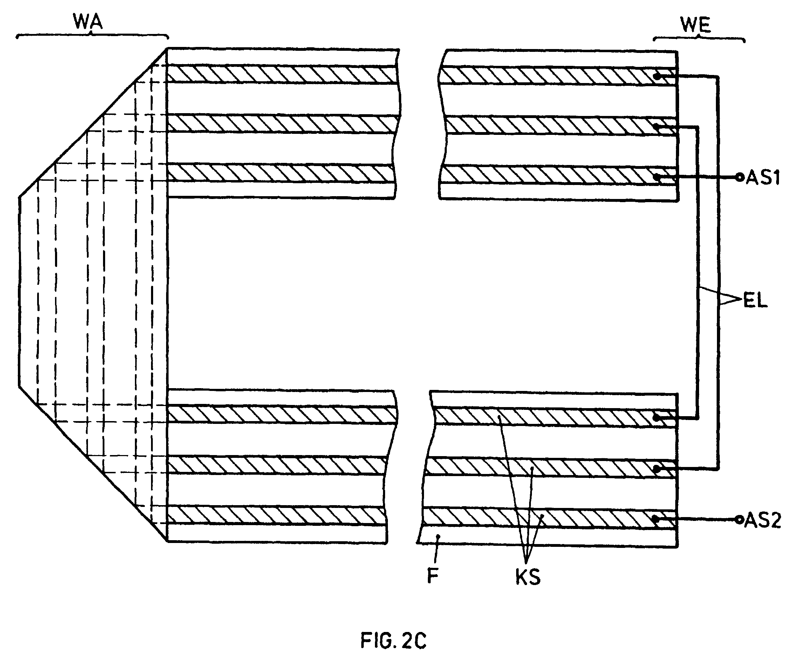

Eine erste Ausführungsform der Induktionsspule wird in Fig. 2 dargestellt, wobei bei dieser anstelle eines Drahtes eine Folie F, die mit parallelen Kupferstreifen KS beschichtet ist, verwendet wird. Die Folienbreite entspricht der gewünschten Wicklungslänge und somit der Länge eines zu bewickelnden Spulenkörpers SK. Die Folie F wird, wie in Fig. 2A dargestellt, direkt auf den Spulenkörper SK gewickelt, wobei die gesamte Drahtlage einer herkömmlichen Induktionsspule einer Folienlage entspricht. Aus Fig. 2 B ist ersichtlich, wie die parallelen Kupferstreifen KS über eine elektrische Verbindungsleitung EL miteinander verbunden sind. Dabei ist das jeweils am Wicklungsanfang WA liegende Ende eines Kupferstreifens KS mit einem am Wicklungsende WE liegenden Ende eines Kupferstreifens KS zu verbinden, was allerdings eine relativ komplizierte Verbindungstechnik erfordert.

Aus diesem Grund wurde für den Fall, bei dem lediglich das Differenzsignal von zwei Induktionsspulen benötigt wird, die eben erläuterte Induktionsspule dahingehend verbessert, dass die Anwendung der obengenannten Verbindungstechnik vermieden werden kann. Wie erwähnt, ist bei der vorliegenden Anwendung (Gradiometer), nämlich das Messen von Feldableitungen (Feldgradienten), nur das Differenzsignal von zwei Induktionsspulen von Interesse. Aufgrund dieser Einschränkung kann die Wicklung somit dahingehend vereinfacht werden, indem beide Induktionsspulen aus derselben Folie F angefertigt werden. Dies wird anhand Fig. 2C dargestellt, aus der ersichtlich ist, dass die Folie F am Wicklungsanfang WA zweimal rechtwinklig gefaltet ist. Ferner werden beide Induktionsspulen parallel auf denselben Spulenkörper gewickelt. Dadurch wird erreicht, dass derselbe Kupferstreifen einmal von aussen nach innen und für die zweite Induktionsspule von innen nach aussen geführt wird. Damit wird ein in der ersten Induktionsspule induziertes Signal mit umgekehrter Polarität auch in die zweite Induktionsspule induziert, womit sich gleiche Signale gegenseitig kompensieren. Daraus folgt, dass an den Anschlussstellen AS1 und AS2 bei der in Fig. 2C dargestellten Ausführungsform lediglich das Differenzsignal ansteht. Die anhand Fig. 1 erläuterten und als Differenzverstärker ausgebildeten Verstärkungseinheiten D1 und D2 sind diesfalls nicht mehr notwendig.

Als wesentlicher Vorteil der anhand Fig. 2C erläuterten Induktionsspulen gegenüber den anhand Fig. 2A erläuterten Induktionsspulen ist, dass keine zusätzlichen elektrischen Verbindungsleitungen zwischen dem Wicklungsanfang WA und dem Wicklungsende WE notwendig sind, denn nunmehr befinden sich alle nötigen Verbindungen am Wicklungsende WE (Fig. 2C).

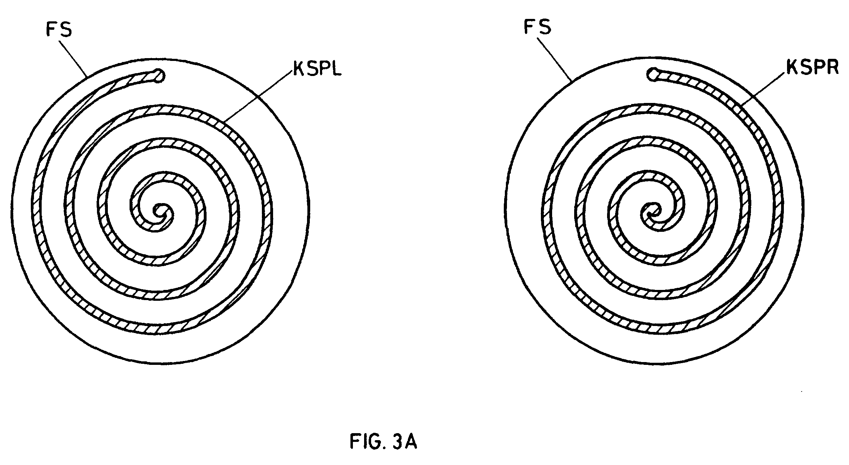

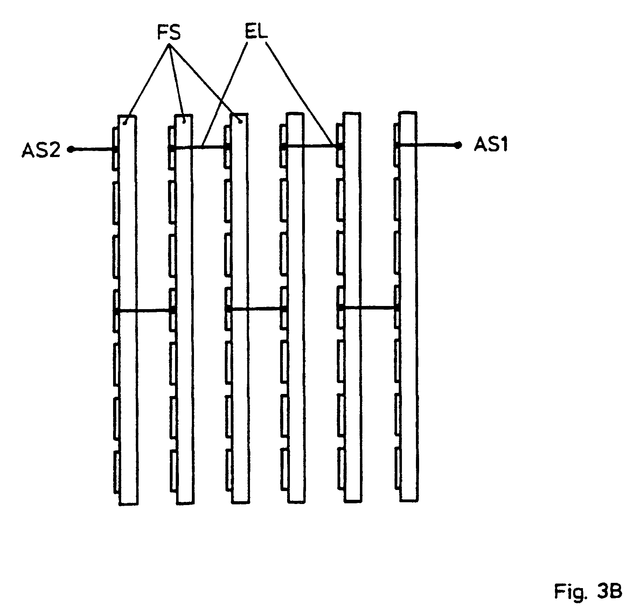

Eine weitere, erfindungsgemässe Ausführungsform der Induktionsspulen ist in Fig. 3 bzw. in Fig. 3A und 3B dargestellt, wobei bei dieser Ausführungsform die Induktionsspulen aus Folienscheiben FS aufgebaut sind, auf die vorzugsweise mittels einem photolithographischen Verfahren entweder eine links- oder eine rechtsdrehende Kupferspirale KSPL bzw. KSPR aufgetragen sind (Fig. 3A). Je eine dieser linksdrehenden Kupferspiralen KSPL ist mit einer rechtsdrehenden Kupferspirale KSPR jeweils im inneren Spiralenanfang über eine elektrische Verbindungsleitung EL verbunden. Dadurch wird erreicht, dass sich der Drehsinn des Kupferstreifens vom äusseren Anfang der linksdrehenden Spirale zum äusseren Ende der rechtsdrehenden Spirale nicht ändert. Dies bedeutet, dass sich die induzierten Signale addieren. Die Induktionsspule wird nun aus einem Stapel solcher links- und rechtsdrehender Spiralenpaare entsprechend der in Fig. 3B dargestellten Anordnung aufgebaut, wobei die Spiralenpaare nur aussen elektrisch verbunden werden müssen.

Die anhand Fig. 2 und 3 erläuterten Induktionsspulen können in jedweden Anwendungen verwendet werden, in denen Magnetfeldkomponenten oder deren Ableitungen bestimmt werden müssen. Insbesondere wenn eine hohe Genauigkeit der zu messenden Grössen und eine hohe Sensitivität der Messeinheit verlangt wird, eignen sich die angegebenen Induktionsspulen besonders als Gradiometer. Darüber hinaus drängen sich die derart ausgebildeten Induktionsspulen geradezu zur Bestimmung von kleinen Magnetfeldkomponenten bzw. deren Ableitungen auf, denn kleine Werte können mit diesen Induktionsspulen äusserst genau gemessen werden. Ferner gewährleistet auch eine einfache Herstellung derartiger Induktionsspulen eine überaus gute Reproduzierbarkeit der gemessenen Grössen mit verschiedenen Induktionsspulen.

Da die als Empfangseinheit S11 bis S22 verwendeten Induktionsspulen nur auf zeitlich sich ändernde Felder reagieren, wird die als Sendeeinheit SE ausgebildete Sendespule mit einem zeitlich variablen Stromsignal von bekannter Form und Grösse angeregt.

Als Empfangseinheit S11 bis S22 können aber auch andere Sensoren als Induktionsspulen verwendet werden. Denkbar ist insbesondere die Verwendung von SQUID ("Sensors", W. Göpel et al. Verlag VC Hauer, Weinheim, 1989).

Die Positionsbestimmung einer Sendeeinheit SE durch Messung des von ihr erzeugten modulierten Feldes mit Hilfe von Gradiometern ist nicht beschränkt auf die Positionsbestimmung von zu bestrahlenden Tumoren. Vielmehr lässt sich die erfindungsgemässe Vorrichtung überall dort erfolgreich einsetzen, wo eine berührungsfreie Positionsbestimmung benötigt wird.

Bei der erläuterten Ausführungsvariante mit einer Sendeeinheit beim Objekt, dessen Position bestimmt werden soll, ist in einer Weiterentwicklung denkbar, dass weitere Sendeeinheiten beim jeweiligen Objekt oder anderen Orten plaziert werden. Damit können mit den Empfangseinheiten mehrere Positionen bestimmt werden. Es muss allerdings darauf geachtet werden, dass die Sendeeinheiten entweder in verschiedenen Zeitabschnitten (Zeitmultiplexverfahren) oder mit verschiedenen Frequenzen (Frequenzmultiplexverfahren) senden.

In ähnlicher Weise ist auch die Ausführungsvariante, bei der eine Empfangseinheit im bzw. in der Nähe des Objektes vorgesehen ist, derart weiter entwickelbar, dass weitere Empfangseinheiten im bzw. in der Nähe des Objektes oder an anderen Stellen vorgesehen sind. Vorteilhaft bei dieser Ausführungsform ist dabei, dass die Positionen der verschiedenen Empfangseinheiten simultan bestimmt werden können, da weder ein Zeitmultiplex- noch ein Frequenzmultiplexverfahren notwendig ist.

Die erfindungsgemässe Vorrichtung wurde anhand einer medizinischen Anwendung ausführlich erläutert. Dadurch wird die Universalität der Erfindung jedoch in keiner Weise eingeschränkt. So eignet sich die erfindungsgemässe Vorrichtung insbesondere als sogenannte dreidimensionale Maus in "Cyber Space"- Anwendungen oder dergleichen.

**Patentansprüche**

1. Vorrichtung zur Bestimmung der Position eines Objektes (T) in einem Raum (P), insbesondere zur Bestimmung eines Tumors in einem menschlichen Körper, wobei mehrere Empfangseinheiten (S11, ..., S22) und eine Sendeeinheit (SE) vorgesehen sind, **dadurch gekennzeichnet, dass** mindestens zwei Empfangseinheiten (S11, ... S22) für jeden zu bestimmenden Parameter vorgesehen sind, der einem Freiheitsgrad der Sendeeinheit (SE) entspricht, dass die zwei zur Bestimmung eines solchen Parameters benötigten Empfangseinheiten (S11, ..., S22) zur Messung einer Feldableitung zusammen geschaltet sind, wobei die Sendeeinheit (SE) im oder möglichst nahe beim zu beobachtenden Objekt (T) und die Empfangseinheiten (S11, ..., S22) vorzugsweise ausserhalb des Raumes (P) positioniert sind.

**2.** Vorrichtung zur Bestimmung der Position eines Objektes (T) in einem Raum (P), insbesondere zur Bestimmung eines Tumors in einem menschlichen Körper, wobei mehrere Sendeeinheiten (S11, ..., S22) und eine Empfangseinheit (SE) zum Senden bzw. Empfangen eines magnetischen Feldes vorgesehen sind, **dadurch gekennzeichnet, dass** zwei Sendeeinheiten (S11, ..., S22) für jeden zu bestimmenden Parameter vorgesehen sind, der einem Freiheitsgrad der Empfangseinheit (SE) entspricht, dass die zwei zur Bestimmung eines solchen Parameters benötigten Sendeeinheiten (S11, ..., S22) zur Erzeugung eines Differenzfeldes zusammen geschaltet sind, wobei die Empfangseinheit (SE) im oder möglichst nahe beim zu beobachtenden Objekt (T) und die Sendeeinheiten (S11, ..., S22) vorzugsweise ausserhalb des Raumes (P) positioniert sind.

**3.** Vorrichtung nach Anspruch 1 oder 2, **dadurch gekennzeichnet, dass** die Sendeeinheit (SE; S11, ..., S22) und/oder die Empfangseinheit (S11, ..., S22; SE) einen Energiespeicher aufweisen.

**4.** Vorrichtung nach Anspruch 1 oder 2, **dadurch gekennzeichnet, dass** ausserhalb des Raumes (P) eine Generatoreinheit (GE) vorgesehen ist, wobei die Generatoreinheit (GE) mit der im Raum (P) positionierten Sendeeinheit bzw. Empfangseinheit (SE) wirkverbunden ist.

**5.** Vorrichtung nach einem der vorangehenden Ansprüche, **dadurch gekennzeichnet, dass** mindestens eine Kalibrierspule mit bekannter Position zum Kalibrieren des Messresultate vorgesehen sind.

**6.** Vorrichtung nach einem der vorangehenden Ansprüche, **dadurch gekennzeichnet, dass** die Empfangseinheiten (SE; S11, ..., S22) als Induktionsspulen ausgebildet sind, dass die Wicklungen der Induktionsspulen als auf einer Folie (F) aufgetragene parallele Streifen (KS), die elektrisch leidend sind, ausgebildet sind und dass die Enden der parallelen Streifen (KS) mittels elektrischen Verbindungsleitungen (EL) zu Wicklungen verbunden sind.

**7.** Vorrichtung nach Anspruch 6, **dadurch gekennzeichnet, dass** die Folie (F) zweimal zur Bildung von zwei miteinander verbundenen, identischen Induktionsspulen gefaltet ist.

**8.** Vorrichtung nach einem der Ansprüche 1 bis 5, **dadurch gekennzeichnet, dass** die Empfangseinheiten (SE; S11, ..., S22) als Induktionsspulen ausgebildet sind, dass die Induktionsspulen aus mehreren Folienscheiben (FS) bestehen und dass auf die Folienscheiben (FS) elektrisch leitende Spiralen (KSPL, KSPR) aufgetragen sind.

**9.** Vorrichtung nach Anspruch 8, **dadurch gekennzeichnet, dass** jeweils eine rechts- und eine linksdrehende Spirale (KSPL, KSPR) zu einem Spiralenpaar am inneren Spiralenende miteinander elektrisch verbunden sind und dass die Spiralenpaare am äusseren Spiralenende miteinander verbunden sind.

**10.** Vorrichtung nach einem der Ansprüche 1 bis 5, **dadurch gekennzeichnet, dass** die Empfangseinheiten (SE; S11, ..., S22) als Magnetfeldsensoren ausgebildet sind, die insbesondere vom Typ SQUID sind.

**11.** Vorrichtung nach einem der vorangehenden Ansprüche, **dadurch gekennzeichnet, dass** eine Signalaufbereitungseinheit (SA) und eine Recheneinheit (RE) vorgesehen sind, dass die mit den Empfangseinheiten (SE; S11, ..., S22) gemessenen Signale der Signalaufbereitungseinheit (SA) und die in der Signalaufbereitungseinheit (SA) aufbereiteten Signale der Recheneinheit (RE) zur Bestimmung der Position beaufschlagt sind.

**12.** Vorrichtung nach einem der vorangehenden Ansprüche, **dadurch gekennzeichnet, dass** eine Bestrahlungseinheit (BE) zur Bestrahlung des Objektes (T) vorgesehen ist.

**13.** Vorrichtung nach Anspruch 12, **dadurch gekennzeichnet, dass** die Bestrahlungseinheit (BE) dem Objekt (T) nachführbar ist oder dass die Bestrahlungseinheit (BE) lediglich dann aktiviert wird, wenn sich das Objekt (T) in einem fix vorgebbaren Zielbereich befindet.

**14.** Verwendung der Vorrichtung nach einem der Ansprüche 1 bis 11 für dreidimensionale Positionseingabeeinheiten.

**15.** Verfahren zur Bestimmung der Position eines Objektes (T) in einem Raum (P), insbesondere zur Bestimmung eines Tumors in einem menschlichen Körper, wobei das Verfahren darin besteht,

- dass mindestens eine Sendeeinheit (SE) im Objekt (T) oder möglichst nahe beim Objekt (T) positioniert wird,

- dass die Sendeeinheit (SE) ein elektromagnetisches Feld, vorzugsweise ein magnetisches Feld, erzeugt und

- dass aufgrund von Feldableitungsmessungen die Position der Sendeeinheit (SE) bestimmt wird.

16. Verfahren zur Bestimmung der Position eines Objektes (T) in einem Raum (P), insbesondere zur Bestimmung eines Tumors in einem menschlichen Körper, wobei das Verfahren darin besteht,

- dass mindestens eine Empfangseinheit (SE) im Objekt (T) oder möglichst nahe beim Objekt (T) positioniert wird,

- dass mindestens zwei ausserhalb des Raumes (P) liegende Sendeeinheiten (S11, ..., S22) ein magnetisches Differenzfeld erzeugen und

- dass aufgrund von Messungen des elektromagnetischen Feldes in der Empfangseinheit (SE) die Position der Empfangseinheit (SE) bestimmt wird.

**Claims**

1. Device for determining the position of an object (T) in a space (P), in particular for determining a tumour in a human body, where several receiver units (S11, ..., S22) and a transmitter unit (SE) are provided, **characterised in that** at least two receiver units (S11, ...., S22) are provided for each parameter to be determined which corresponds to a degree of freedom of the transmitter unit (SE), that the two receiver units (S11, ...., S22) required to determine such a parameter arc connected together to measure a field gradient, where the transmitter unit (SE) is positioned in or as close as possible to the object to be observed (T) and the receiver units (S11, ....., S22) are preferably positioned outside the space (P).

2. Device for determining the position of an object (T) in a space (P), in particular for determining a tumour in a human body, where several transmitter units (S11, ...., S22) and a receiver unit (SE) are provided for sending and receiving a magnetic field. **characterised in that** two transmitter units (S11, ....., S22) are provided for each parameter to be determined which corresponds to a degree of freedom of the receiver unit (SE), that the two transmitter units (S11, ....., SE22) required to determine such a parameter are connected together to generate a differential field, where the receiver unit (SE) is positioned in or as close as possible to the object to be observed (T) and the transmitter units (SE11, ....., SE22) are preferably positioned outside the space (P).

3. Device according to claim 1 or 2, **characterised in that** the transmitter unit (SE; S11, ....., S22) and/or the receiver unit (SE11, ....., SE22; SE) have an energy accumulator.

4. Device according to claim 1 or 2, **characterised in that** a generator unit (GE) is provided outside the space (P), where the generator unit (GE) is actively connected with the transmitter unit or receiver unit (SE) positioned inside the space (P).

5. Device according to any of the previous claims, **characterised in that** at least one calibration coil with known position is provided to calibrate the measurement results.

6. Device according to any of the previous claims, **characterised in that** the receiver units (SE; SE11, ....., SE22) are formed as induction coils, that the windings of the induction coils are formed as parallel electrically conductive strips (KS) applied to a film (F) and that the ends of the parallel strips (KS) are connected to windings by means of electrical connecting leads (EL).

7. Device according to claim 6, **characterised in that** the film (F) is folded twice to form two interconnected identical induction coils.

8. Device according to any of claims 1 to 5, **characterised in that** the receiver units (SE; SE11, ....., SE22) are formed as induction coils, that the induction coils consist of several film discs (FS) and that electrically conductive spirals (KSPL, KSPR) are applied to the film discs (FS).

9. Device according to claim 8, **characterised** that in each case a right- and a left-turning spiral (KSPL, KSPR) are connected together electrically at the inner spiral ends into a spiral pair and that the spiral pairs are connected together at the outer spiral ends.

10. Device according to any of claims 1 to 5, **characterised in that** the receiver units (SE; SE11, ....., SE22) are formed as magnetic field sensors, in particular of the SQUID type.

11. Device according to any of the previous claims, **characterised in that** a signal preparation unit (SE) and a computer unit (RE) are provided which are exposed to the signals from the signal preparation unit (SA) measured by the receiver units (SE; SE11, ....., SE22) and the signals prepared in the signal preparation unit (SE) from the computer unit (RE) to determine the position.

12. Device according to any of the previous claims, **characterised in that** an irradiation unit (BE) is provided to irradiate the object (T).

**13.** Device according to claim 12, **characterised in that** the irradiation unit (BE) can be guided according to the object (T) or that the irradiation unit (BE) is activated only when the object (T) is in a fixed prespecifiable target area.

**14.** Use of the device according to any of claims 1 to 11 for three dimensional position input units.

**15.** Process for determining the position of an object (T) in a space (P), in particular for determining a tumour in a human body, where the process comprises:

- at least one transmitter unit (SE) is positioned in the object (T) or as close as possible to the object (T),
- the transmitter unit (SE) generates an electromagnetic field, preferably a magnetic field, and
- the position of the transmitter unit (SE) is determined from field gradient measurements.

**16.** Process for determining the position of an object (T) in a space (P), in particular for determining a tumour in the human body, where the process comprises:

- at least one receiver unit (SE) is positioned in the object (T) or as close as possible to the object (T),
- at least two transmitter units (SE11, ....., SE22) lying outside the space (P) generate a magnetic differential field and
- the position of the receiver unit (SE) is determined on the basis of measurements of the electromagnetic field in the receiver unit (SE).

**Revendications**

**1.** Dispositif pour définir la position d'un objet (T) dans un espace (P), en particulier pour définir une tumeur dans un corps humain, plusieurs unités de réception (S11, ..., S22) et une unité d'émission (SE) étant prévues, **caractérisé en ce qu'**il est prévu au moins deux unités de réception (S11, ..., S22) pour chaque paramètre à définir qui correspond à un degré de liberté de l'unité d'émission (SE), et **en ce que** les deux unités de réception (S11, ..., S22) nécessaires pour définir un tel paramètre sont interconnectées pour mesurer une dérivation de champ, l'unité d'émission (SE) étant positionnée dans l'objet à observer (T) ou le plus près possible de celui-ci, tandis que les unités de réception (S11, ..., S22) sont positionnées de préférence à l'extérieur de l'espace (P).

**2.** Dispositif pour définir la position d'un objet (T) dans un espace (P), en particulier pour définir une tumeur dans un corps humain, plusieurs unités d'émission (S11, ..., S22) et une unité de réception (SE) étant prévues pour émettre et recevoir un champ magnétique, **caractérisé en ce qu'**il est prévu deux unités d'émission (S11, ..., S22) pour chaque paramètre à définir, qui correspond à un degré de liberté de l'unité de réception (SE), et **en ce que** les deux unités d'émission (S11, ..., S22) nécessaires pour définir un tel paramètre sont interconnectées pour générer un champ différentiel, l'unité de réception (SE) étant positionnée dans l'objet à observer (T) ou le plus près possible de celui-ci, tandis que les unités d'émission (S11, ..., S22) sont positionnées de préférence à l'extérieur de l'espace (P).

**3.** Dispositif selon la revendication 1 ou 2, **caractérisé en ce que** l'unité d'émission (SE ; S11, ..., S22) et/ou l'unité de réception (S11, ..., S22 ; SE) comportent une réserve d'énergie.

**4.** Dispositif selon la revendication 1 ou 2, **caractérisé en ce qu'**il est prévu à l'extérieur de l'espace (P) une unité génératrice (GE), cette unité génératrice (GE) étant en relation fonctionnelle avec l'unité d'émission ou l'unité de réception (SE) positionnée dans l'espace (P).

**5.** Dispositif selon l'une des revendications précédentes, **caractérisé en ce qu'**il est prévu au moins une bobine de calibrage avec une position connue, pour calibrer les résultats de mesure.

**6.** Dispositif selon l'une des revendications précédentes, **caractérisé en ce que** les unités de réception (SE ; S11, ..., S22) sont conçues comme des bobines d'induction, **en ce que** les enroulements des bobines d'induction sont conçus comme des bandes parallèles (KS) appliquées sur un film (F) et conductrices d'électricité, et **en ce que** les extrémités des bandes parallèles (KS) sont reliées à l'aide de conduites de raccordement électriques (EL) pour former des enroulements.

**7.** Dispositif selon la revendication 6, **caractérisé en ce que** le film (F) est plié deux fois pour former deux bobines d'induction identiques reliées entre elles.

**8.** Dispositif selon l'une des revendications 1 à 5, **caractérisé en ce que** les unités de réception (SE ; S11, ..., S22) sont conçues comme des bobines d'induction, **en ce que** les bobines d'induction se composent de plusieurs plaques en forme de films (FS) et **en ce que** des spirales (KSPL, KSPR) conductrices d'électricité sont appliquées sur les plaques en forme de films (FS).

**9.** Dispositif selon la revendication 8, **caractérisé en ce qu'**une spirale tournant à droite et une spirale tournant à gauche (KSPL, KSPR) sont reliées électriquement, au niveau de leur extrémité intérieure, pour former une paire de spirales, et **en ce que** les paires de spirales sont reliées entre elles au niveau de l'extrémité de spirale extérieure.

10. Dispositif selon l'une des revendications 1 à 5, **caractérisé en ce que** les unités de réception (SE ; S11, ..., S22) sont conçues comme des capteurs de champ magnétique, qui sont en particulier du type SQUID.

11. Dispositif selon l'une des revendications précédentes, **caractérisé en ce qu'**il est prévu une unité de traitement de signaux (SA) et une unité de calcul (RE), et **en ce que** les signaux de l'unité de traitement de signaux (SA) mesurés avec les unités de réception (SE ; S11, ..., S22), et les signaux de l'unité de calcul (RE) traités par ladite unité de traitement (SA) sont utilisés pour définir la position.

12. Dispositif selon l'une des revendications précédentes, **caractérisé en ce qu'**il est prévu une unité d'irradiation (BE) pour irradier l'objet (T).

13. Dispositif selon la revendication 12, **caractérisé en ce que** l'unité d'irradiation (BE) peut suivre l'objet (T) ou **en ce que** qu'elle est activée simplement lorsque l'objet (T) se trouve dans une zone cible apte à être prédéfinie de manière fixe.

14. Utilisation du dispositif selon l'une des revendications 1 à 11 pour des unités d'entrée de position à trois dimensions.

15. Procédé pour définir la position d'un objet (T) dans un espace (P), en particulier pour définir une tumeur dans un corps humain, selon lequel

  - au moins une unité d'émission (SE) est positionnée dans l'objet (T) ou le plus près possible de l'objet (T),
  - l'unité d'émission (SE) génère un champ électromagnétique, de préférence un champ magnétique, et
  - à partir des mesures de dérivation de champ, on définit la position de l'unité d'émission (SE).

16. Procédé pour définir la position d'un objet (T) dans un espace (P), en particulier pour définir une tumeur dans un corps humain, selon lequel

  - au moins une unité de réception (SE) est positionnée dans l'objet (T) ou le plus près possible de l'objet (T),
  - au moins deux unités d'émission (S11, ..., S22) situées à l'extérieur de l'espace (P) génèrent un champ différentiel magnétique, et
  - à partir des mesures du champ électromagnétique dans l'unité de réception (SE), on définit la position de l'unité de réception (SE).

FIG.1

FIG. 2A

FIG. 2B

FIG. 2C

FIG. 3A

Fig. 3B